# EUROPEAN PATENT APPLICATION

(11) **EP 4 552 576 A1**
(43) Date of publication of application: **14.05.2025**
(21) Application number: 23209102.5
(22) Date of filing: 10.11.2023
(51) Int. Cl.: A61B 6/00, G06T 11/00, A61C 9/00, G06T 7/30, G06T 3/14, A61B 5/00, A61B 6/58, G06T 3/20, G06T 3/40, G06T 3/4076, G06T 3/60

(54) **A METHOD AND SYSTEM FOR REDUCING POSITION ARTEFACTS IN DENTAL PANORAMIC IMAGES**

(71) Applicant: Newton2 ApS, 2730 Herlev (DK)
(72) Inventor: ALLIN, Thomas Højgaard, 2730 Herlev (DK); SØRENSEN, Thomas Sangild, 2730 Herlev (DK); RIIS, Nicolai André Brogaard, 2730 Herlev (DK)
(74) Representative: Guardian IP Consulting I/S

(57) **Abstract**

Presented is a method for producing a dental panoramic image, the method comprising: recording one or more images during a panoramic scan of a patient, providing an image data input to a trained machine learning model, the image data input being based on the recorded one or more images and the image data input being indicative of patient positioning during the panoramic scan, generating a positioning parameter set by the trained machine learning model, the positioning parameter set being based on the received image data input, where the positioning parameter set is representative of the position of the patient's head during the panoramic scan, reconstructing one or more corrected-positioning dental panoramic images based on x-ray frame data recorded during the panoramic scan and on the positioning parameter set. Further, an x-ray imaging system and a computer-implemented method.

## Description

The present disclosure relates to a method and system for reducing artefacts in dental panoramic images created from x-ray data. In particular, the disclosure relates to compensation for incorrect patient positioning during acquisition of the x-ray data such as compensation for e.g. tilting, rotation, and/or translation of the patient's head during recording of the x-ray data.

### Background

Various x-ray scanners may be used to obtain dental panoramic images, also referred to as dental panoramic x-ray radiographs or orthopantomograms. A dental panoramic image ideally shows a patient's entire mouth in a single image, including the teeth, upper and lower jaws, surrounding structures and tissues, and is used in the diagnosis and treatment of dental and medical conditions.

A dental panoramic image is reconstructed by "compositing" (e.g. reconstruction by back projection) from hundreds to several thousand narrow x-ray projections and seeks to provide a broad view of the entire dental arch.

In order to obtain x-ray data of sufficient quality for panoramic images it is important to position the patient correctly in the x-ray scanner prior to image acquisition as any discrepancy between the expected and actual patient positioning results in image degradation. Examples of such image degradation due to incorrect patient positioning are e.g. right/left asymmetry, unintended image x-ray shadows, geometric and anatomical distortions and out-of-focus depiction. Further, even after positioning the patient needs to remain motionless during the entire subsequent x-ray scan acquisition in order to obtain optimal image quality and any movement during acquisition will likely generate one or more of the mentioned artefacts.

In order to reduce incorrect patient positioning and motion during acquisition, many x-ray scanners include positioning and/or fixation means. However, non-restraining positioning means such as positioning lasers and chin rests do not prevent the patient from moving after being positioned. Further, many patients find fixation means discomforting, which may cause a patient to either forego having the x-ray scan performed or to react to the restraints in such a way as to position themselves incorrectly for the scan or to move during the x-ray data acquisition.

Despite detailed guidelines on how to correctly position a patient in the scanner and despite efforts on the part of the technician to follow the guidelines encountering cases with minor or major degrees of head misalignment is not rare.

Thus, there is a need for an improved method for producing a dental panoramic image and x-ray imaging system. It is an object to provide an improved method and system for producing a dental panoramic image.

It is a further object to provide a method and x-ray imaging system, which compensates for incorrect patient positioning during acquisition of x-ray data, in particular during acquisition of x-ray data used for reconstruction of dental panoramic images.

It is a further object to facilitate the provision of high quality dental panoramic images.

### Summary

The present disclosure relates to different aspects each yielding one or more of the benefits and advantages described in connection with one or more of the other aspects, and each having one or more embodiments corresponding to the embodiments described in connection with one or more of the other aspects and/or disclosed in the appended claims. In the aspects, the terms and features relate to the terms and features having the same name in the other aspects and therefore the descriptions and explanations of terms and features given in one aspect apply to the other aspects.

According to an aspect, disclosed herein are embodiments of a method for producing a dental panoramic image, the method comprising:
- recording one or more images during a panoramic scan of a patient,
- providing an image data input to a trained machine learning model, the image data input being based on the recorded one or more images and the image data input being indicative of patient positioning during the panoramic scan,
- generating a positioning parameter set by the trained machine learning model, the positioning parameter set being based on the received image data input, where the positioning parameter set is representative of the position of the patient's head during the panoramic scan, and
- reconstructing one or more corrected-positioning dental panoramic images based on x-ray frame data recorded during the panoramic scan and on the positioning parameter set.

A dental panoramic image, which may also be referred to as a dental panoramic x-ray radiograph or an orthopantomogram, is a two-dimensional curved x-ray image following the shape of the mandible. Conceptually, the image curvature can be described by a curve in a transverse plane of the patient and this curve spans the horizontal axis in the two-dimensional panoramic image. The vertical image axis is defined as the patient's feet/head axis, which is perpendicular to the curve in the transverse plane. Ideally, the central curve segment follows the shape of the tooth line, while both distal curve segments follow the shape of the jaw and temporomandibular joint.

A panoramic scan is an x-ray scan during which x-ray frame data suitable for construction of a dental panoramic image are acquired. A dental panoramic image may be described as a two-dimensional, wide-angle view of a patient's oral and maxillofacial region, including the teeth, jaws, and surrounding structures. A panoramic scan may be performed using any suitable x-ray apparatus, such as e.g. a dedicated panoramic scanner, a panoramic and cephalometric dental x-ray machine, a CBCT scanner with a panoramic modality, or any other x-ray apparatus with a panoramic modality, i.e. a modality wherein the apparatus is configured to provide one or more dental panoramic images based on acquired x-ray frame data.

The recorded one or more images, i.e. the one or more images that are recorded during a panoramic scan of a patient, may be one or more of: one or more optical images, and/or one or more x-ray images, such as one or more projection x-ray images. The one or more x-ray images may be images based on x-ray frame data outputted from an x-ray detector. The recorded one or more images may be extra-oral images, i.e. images recorded using an image sensor/detector that is entirely outside of the patient's mouth during the recording of the one or more images.

The image data input may be x-ray images, reconstructed x-ray images, optical images, or other types of image data. Thus, the image data input may be of the same type as the one or more images recorded during the panoramic scan, or the recorded one or more images may have been processed to provide the image data input. For example, the recorded one or more images may be one or more x-ray images, which are then processed to produce a reconstructed x-ray image that is subsequently provided to the machine learning model as image data input. Thus, in some embodiments, the image data input comprises one or more of: one or more optical images, and/or one or more x-ray images, such as one or more projection x-ray images, and/or one or more reconstructed images, such as one or more reconstructed panoramic images.

Reconstruction of a dental panoramic image refers to the process of creating a single two-dimensional (2D) dental panoramic image from a series of 2D X-ray projection images taken at different angles. The panoramic image is reconstructed by "compositing" (e.g. reconstruction by back projection) from hundreds to several thousand narrow x-ray projections. As an example, current dedicated digital x-ray detectors typically record 2D projections of 6 mm in width and a height of 15 cm at a high frame rate. Data acquisition lasts 10-20 seconds during which an x-ray tube and detector pair rotates and translates around the patient; a rotational movement around the patient's feet/head axis is combined with a translational movement along the patient's anterior/posterior axis. These motor movements result in an elliptical displacement trajectory of the x-ray tube and detector during acquisition. It should be noted that this elliptical "motor trajectory" is defined independently of the curved shape of the image plane underlying the resulting image composition as is well known to the skilled person.

In some embodiments, the one or more recorded images comprises one or more optical images. The one or more optical images may be recorded using visible light, IR, and/or UV light. The one or more optical images may be recorded using one or more image sensors, such as one or more cameras, such as one or more digital cameras. The one or more optical images may be recorded using one or more high-resolution image sensors, such as one or more high-resolution digital cameras. The one or more optical images may be one or more single images and/or a time sequence of optical images/optical video frames. The time sequence of optical images may be configured in a video format. The time sequence may be a high-speed video clip.

In some embodiments, the one or more recorded images comprises one or more x-ray images. The one or more x-ray images may be projection x-ray images, i.e. x-ray images obtained by projection imaging. The one or more projection x-ray images may be obtained using an x-ray apparatus, such as e.g. a dedicated panoramic scanner, a combined panoramic and cephalometric dental x-ray machine, a CBCT scanner, or any other x-ray apparatus that is configured to record projection x-ray images. The one or more x-ray images may be, or be based on, x-ray frame data recorded during the panoramic scan. The x-ray frame data used as image data input indicative of patient positioning may at least in part be x-ray frame data that is also used in the reconstruction of the one or more corrected-positioning dental panoramic images.

In some embodiments, the one or more recorded images comprises one or more reconstructed dental panoramic images. The one or more reconstructed images may be one or more reconstructed dental panoramic images. In some embodiments, the method further comprises generating an initial dental panoramic image based on x-ray frame data recorded during the panoramic scan, and the step of providing the image data input to a trained machine learning model comprises providing the initial dental panoramic image as image data input to the trained machine learning model. In some embodiments, the initial dental panoramic image has a lower resolution compared to the resolution of the one or more corrected-positioning dental panoramic images. That is, the trained machine learning model may generate the positioning parameter set based on received one or more initial dental panoramic images, possibly of a lower resolution compared to the resolution of the final panoramic images, i.e. compared to the resolution of the corrected-positioning dental panoramic images. The positioning parameter set is used in the reconstruction of one or more corrected-positioning dental panoramic images.

The recorded one or more images may be fed directly to the trained machine learning model as image data input. Alternatively, the recorded one or more images may be processed prior to being provided to the trained machine learning model. Processing the recorded one or more images may comprise processing of the recorded raw image(s) such as image enhancement, filtering, rescaling, compression, reconstruction, stitching, etc.

The image data input is indicative of the patient's position, such as the position of the patient's head, during the scan. For example, one or more optical images provided as image data input may show at least part of the patient's head, such as show a frontal view and/or a side view of the patient's head. Raw or processed x-ray frame data may show at least part of the patient's oral and maxillofacial region, for example teeth, jaws, and surrounding structures.

The trained machine learning model is trained to generate a positioning parameter set based on received image data input. The trained machine learning model may be a trained neural network. The trained machine learning model, in particular a trained neural network model, may be trained to estimate the positioning of the patient's head, and to do so based on the received image data input. The trained machine learning model is configured to generate an output, the positioning parameter set, which is representative of the estimated position of the patient's head based on the received image data input. The trained machine learning model has been trained to generate a positioning parameter set that is representative of the position of a patient's head based on new, unseen image data input by utilising what it has learned from training data. The trained machine learning model may be trained to generate the positioning parameter set based on different types of image data input and/or based on a plurality of types of image data input. For example, the trained machine learning model may have been trained to generate a positioning parameter set based on one or more optical images, and/or based on one or more raw x-ray frame data and/or based on one or more processed x-ray frame data. Processed x-ray frame data may be one or more reconstructed dental panoramic images. Thus, the trained machine learning model may have been trained to generate a positioning parameter set based on any one of a plurality of types of image data as input, for example on either optical image(s) or raw x-ray frame data or on either optical image(s) or reconstructed panoramic images. Alternatively, or additionally, the trained machine learning model may have been trained to generate a positioning parameter set based on image data input, which comprises a plurality of types of image data as input, for example on image data input comprising both optical image(s) and raw x-ray frame data or both optical image(s) and reconstructed panoramic image(s).

The trained machine learning model may have been trained using any method of training a machine learning model, such as any known method of training a machine learning model. For example, the trained machine learning model may have been trained using any of: supervised, unsupervised, semi-supervised, weakly supervised, or reinforcement learning. Thus, as an example, the trained machine learning model may have been trained using labelled data, where the input data is correlated with target labels or outcomes (supervised learning), i.e. with image data input that has been correlated with a positioning parameter set. As another example, the trained machine learning model may have been trained using unlabelled data, where the algorithm is provided with input data, but not with any explicit outcomes (unsupervised learning).

The recorded one or more images, and/or the image data input, and/or x-ray frame data recorded during the panoramic scan may each be associated with a time stamp. The associated time stamp may be used to correlate recorded image(s), and/or image data input, and/or x-ray frame data.

The one or more corrected-positioning dental panoramic images are reconstructed based on x-ray frame data recorded during the panoramic scan and on the positioning parameter set generated by the trained machine learning model. Thus, when reconstructing the one or more corrected-positioning dental panoramic images the process takes into consideration a determined patient positioning during the acquisition of the x-ray images.

Thus, the method for producing a dental panoramic image seeks to automatically correct for head misalignment during the reconstruction stage without need of user interaction and does so by quantifying the patient positioning, such as orientation of the patient's head, from one or more images captured during the x-ray data acquisition phase, and then using this quantified patient positioning, such as head pose of the patient, as an input to a reconstruction algorithm. The algorithm may then define the curved image plane in three-dimensional space used in reconstruction so as to make it correlate with the quantified patient head position as determined rather than to a predefined expected patient head position.

The quantification of the patient positioning, such as patient head pose, from the image data input is carried out by a trained machine learning model, which has been trained to receive image data input, such as e.g. optical images, and/or raw x-ray frame data, and/or processed x-ray frame data, as input and to output parameters, i.e. the positioning parameter set, describing the patient's determined positioning, such as the patient's head pose. The output parameter set from the machine learning model may comprise a plurality of parameters, such as individual scalar values, which define the patient positioning. In some embodiments, the position of the patient's head during the panoramic scan is defined relative to a pre-defined coordinate system. In some embodiments, the pre-defined coordinate system is a pre-defined head positioning or a pre-defined global coordinate system. A pre-defined head positioning coordinate system may be centred according to an expected head positioning of the patient. In some embodiments, the output parameter set defines a rigid transformation, i.e. describing rotation and position parameters, such as six scalars for six degrees of freedom. In some embodiments, the positioning parameter set may define a non-rigid transformation, e.g. describing scale and/or shear. Thus, in some embodiments, the positioning parameter set is indicative of a rigid transformation, e.g. of tilt, and/or rotation, and/or translation, of the pre-defined coordinate system and/or a non-rigid transformation, such as scaling and/or shear, of the pre-defined coordinate system.

The positioning parameter set may comprise absolute values relative to a global coordinate system or local offsets, for example offsets to a predefined expected value for each parameter. The positioning parameter set is provided as input to a reconstruction algorithm, and the algorithm bases the reconstruction on the positioning parameter set with the aim to compensate for patient positioning during the panoramic scan. The reconstruction algorithm may utilise a pre-defined standard image curve, which is transformed according to the patient's head position, i.e. transformed according to the positioning parameter set. That is, the reconstruction process involves choosing an image curve, which may be a nominal curve, i.e. a standard curve, possibly selected from a set of standard curves, or creating a customized curve, i.e. a curve that attempts to more closely follow the dental arch of the specific patient. Using a pre-defined standard curve generally requires less processor power compared to producing a custom curve, i.e. a curve, which is customized according to the patient's dental arch. The method described herein does not aim to produce a custom curve, but to compensate for incorrect head positioning during the panoramic scan, i.e. to compensate for a head positioning which deviates from a pre-defined expected head positioning. The pre-defined standard curve may be selected from a set of standard curves. A pre-defined set of standard curves may comprise standard curves, which are parametrized according to different patient sizes.

In some embodiments, the positioning parameter set, which are parameters, such as individual scalar values, that define the patient positioning as well as the one or more reconstructed corrected-positioning panoramic images are provided to a user interface and displayed to a user. The user interface may be configured to receive adjustment input, which is representative of a manually adjusted positioning parameter set, i.e. a positioning parameter set, which is based on the adjustment input from a user. In some embodiments, one or more manually-adjusted dental panoramic images are reconstructed based on x-ray frame data recorded during the panoramic scan and on the manually adjusted positioning parameter set.

In some embodiments, multiple dental panoramic images are reconstructed based on x-ray frame data recorded during the panoramic scan and on the positioning parameter set. In some embodiments, the positioning parameter set comprises a plurality of parameters defining the patient positioning, and each of the multiple dental panoramic images is augmenting one or more of the parameters. Thus, the reconstruction may provide a plurality of dental panoramic images each of which are based on a positioning parameter set having one or more parameters that are each varied between the multiple dental panoramic images. For example, multiple dental panoramic images could be reconstructed each being slightly offset along the patient's anterior/posterior axis. The augmented multiple dental panoramic images may be provided to a user interface and displayed to a user. The user interface may be configured to provide the user with a means, such as e.g. a button, or slider, or the like, to change which of the multiple dental panoramic images is displayed or is in focus. The user interface may be configured to display the multiple dental panoramic images one at a time with the order of the images being based on increasing or decreasing value of an augmented parameter. For example, a user may be presented with multiple panoramic images where each of the images is slightly offset along the patient's anterior/posterior axis compared to the previous and following images displayed. This would allow the user to see the multiple dental panoramic images in a manner where the focus is shifted in the anterior/posterior direction, for example to refocus on the roots/crown of an anterior tooth oriented somewhat perpendicular to the individual images.

According to an aspect, disclosed herein is an x-ray imaging system comprising:
- an x-ray detector configured to convert x-rays to digital electric signals pixel by pixel and to output the electric signal as x-ray frame data,
- a data storage unit configured to store the x-ray frame data outputted from the x-ray detector during a scan of a patient,
- a data processing system,
the x-ray imaging system being configured to execute the steps of a method for producing a dental panoramic image as disclosed herein.

According to an aspect, disclosed herein is an x-ray imaging system comprising:
- an x-ray detector configured to convert x-rays to digital electric signals pixel by pixel and to output the electric signal as x-ray frame data,
- a data storage device configured to store the x-ray frame data outputted from the x-ray detector during a panoramic scan of a patient,

wherein the x-ray imaging system further comprises a data processing system comprising a trained machine learning model configured to receive image data input indicative of patient positioning during the scan of the patient, the image data input being based on one or more images recorded during the panoramic scan,
the trained machine learning model being configured to generate a positioning parameter set based on the image data input, where the positioning parameter set is representative of the position of the patient's head during the panoramic scan, and
wherein the data processing system is further configured to reconstruct one or more corrected-positioning dental panoramic images based on x-ray frame data recorded during the panoramic scan and on the positioning parameter set.

The x-ray imaging system may comprise one or more data processors as part of its data processing system. The data processing system may be a local processing system, an external processing system, or a combination of a local and an external processing system. Some or all of the processing steps, such as reconstruction of dental panoramic images, may be performed by a local data processing system, or some or all of the processing steps may be performed by an external data processing system, or the processing steps may be distributed between a local processing system and an external processing system. A local processing system may be one that is part of, or directly coupled to, the device, which houses the x-ray detector. An external processing system may be a stand-alone computer or a system of multiple computers, e.g. a client-server system, a cloud-based system or the like. The trained machine learning model may be comprised in a local processing system, or in an external processing system, or it may be a distributed machine learning model running in series or parallel on both a local and an external processing system.

In some embodiments, the image data input comprises one or more of: one or more projection x-ray images, and/or one or more reconstructed images, such as one or more reconstruction panoramic images, and/or the x-ray imaging system further comprises one or more cameras and the recorded one or more images comprises one or more optical images.

In some embodiments, the position of the patient's head during the panoramic scan is defined relative to a pre-defined coordinate system. In some embodiments, the pre-defined coordinate system is a pre-defined head positioning or a pre-defined global coordinate system. In some embodiments, the positioning parameter set is indicative of a rigid transformation, i.e. rotation and/or translation, of the pre-defined coordinate system and/or of a non-rigid transformation, such as scaling and/or shear, of the pre-defined coordinate system.

In some embodiments, the data processing system is configured to generate an initial dental panoramic image based on x-ray frame data recorded during the panoramic scan, and wherein the image data input provided to the trained machine learning model comprises the initial dental panoramic image. In some embodiments, the initial dental panoramic image has a lower resolution compared to the resolution of the one or more corrected-positioning dental panoramic images.

According to an aspect, disclosed herein is a computer-implemented method for producing a dental panoramic image, the method comprising the steps:
- receiving one or more images recorded during a panoramic scan of a patient,
- providing an image data input to a trained machine learning model, the image data input being based on the recorded one or more images and the image data input being indicative of patient positioning during the panoramic scan,
- generating a positioning parameter set by the trained machine learning model, the positioning parameter set being based on the received image data input, where the positioning parameter set is representative of the position of the patient's head during a panoramic scan,
- reconstructing one or more corrected-positioning dental panoramic images based on x-ray frame data recorded during the panoramic scan and on the positioning parameter set.

According to an aspect, disclosed herein are embodiments of a data processing system configured to perform steps of a computer-implemented method described herein. In particular, the data processing system may have stored thereon program code adapted to cause, when executed by the data processing system, the data processing system to perform the computer-implemented steps of a method described herein. The data processing system may be embodied as a single computer or as a distributed system including multiple computers, e.g. a client-server system, a cloud based system, etc. The data processing system may include a data storage device for storing the computer program and data such as any or all of: x-ray frame data, recorded optical images, image data input, reconstructed images, positioning parameter set, etc. The data processing system may be part of an x-ray imaging system as described herein and be configured to receive recorded images, and/or x-ray frame data, and/or image data input, and/or positioning parameter set. To this end, the data processing system may comprise a suitable wired or wireless communications interface. The data processing system may be configured to provide image data input based on recorded images as described herein. The data processing system may be configured to reconstruct dental panoramic images based on x-ray frame data, such as to reconstruct initial dental panoramic images and/or corrected-positioning dental panoramic images as described herein. The data processing system may comprise the trained machine learning model as described herein. The data processing system may be configured to provide one or more reconstructed corrected-positioning dental panoramic images, which take into account patient positioning, by feeding the image data input based on the recorded one or more images into the machine learning model, receiving from the machine learning model a positioning parameter set and utilising the positioning parameter set to reconstruct one or more dental panoramic images based on x-ray frame data recorded during the panoramic scan and on the positioning parameter set.

According to an aspect, a computer program comprises program code adapted to cause, when executed by a data processing system, the data processing system to perform computer-implemented steps of a method described herein. The computer program may be embodied as a computer-readable medium, such as a CD-ROM, DVD, optical disc, memory card, flash memory, magnetic storage device, floppy disk, hard disk, etc. having stored thereupon the computer program. According to one aspect, a computer-readable medium has stored thereupon a computer program as described herein.

In the aspects disclosed herein, terms and features relate to the terms and features having the same name in the other aspects and therefore the descriptions and explanations of terms and features given in one aspect apply, with appropriate changes, to the other aspects. Additional aspects, embodiments, features and advantages will be made apparent from the following detailed description of embodiments and with reference to the accompanying drawings.

### Brief description of the drawings

Preferred embodiments will be described in more detail in connection with the appended drawings, where:
Fig. 1, 2, and 3 show flow charts of a method according to some embodiments,
Figs. 4A and 4B show a perspective view of parts of an x-ray imaging system according to some embodiments, and
Fig. 5 shows a diagram of at least part of an x-ray imaging system according to some embodiments.

### Detailed description

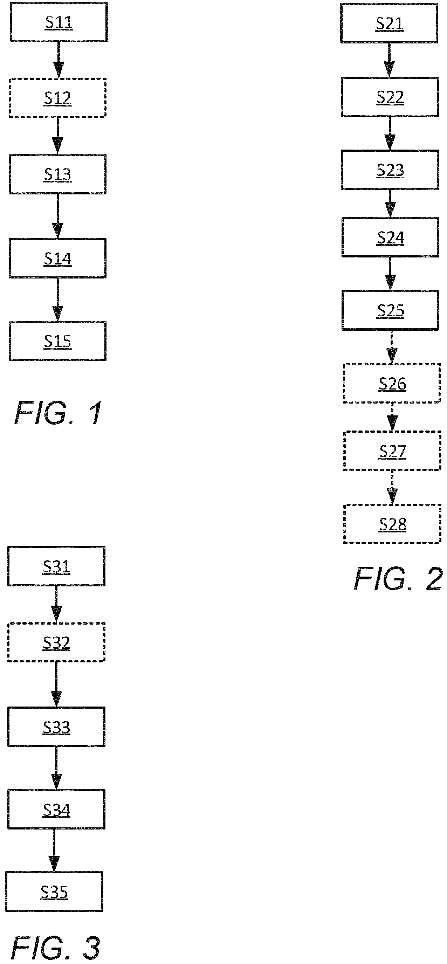

Fig. 1 shows a flow chart of a method for producing a dental panoramic image according to some embodiments.

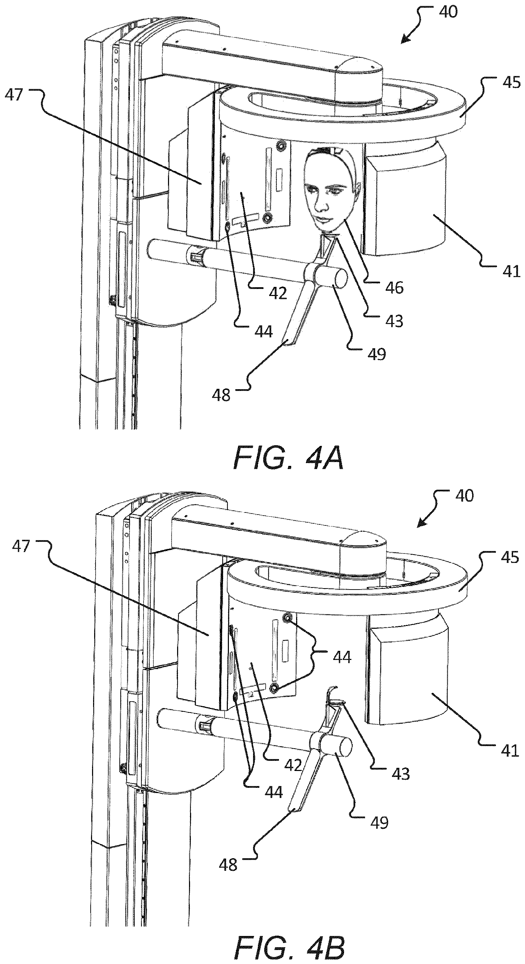

In step S11, one or more optical images are recorded during a panoramic scan of a patient. The one or more optical images may be recorded using one or more cameras attached to the scanner, e.g. as shown in figs. 4A and 4B.

In optional step S12, the recorded one or more optical images are processed . Processing the one or more optical images may involve image processing of the one or more optical images, for example image enhancement, filtering, rescaling, compression, etc. of one or more images.

In step S13, an image data input based on the recorded one or more images is provided to a trained machine learning model, where the image data input is indicative of patient positioning during the panoramic scan. For example, an optical image indicative of the patients positioning may show at least part of the patient's head, such show a frontal view of the patient's head or a side view of the patient's head.

In step S14, the trained machine learning model generates a positioning parameter set being based on the received image data input indicative of patient positioning. The positioning parameter set is representative of the position of the patient's head during the panoramic scan.

In step S15, one or more corrected-positioning dental panoramic images are reconstructed based on x-ray frame data recorded during the panoramic scan and on the positioning parameter set. A corrected-positioning dental panoramic image may be reconstructed using a standard curve, which has been rigidly transformed to correspond to an estimated positioning of the patient's dental curve due to the patient's head being e.g. tilted (looking up or down), and/or rotated (head turned), and/or translated. In some embodiments, a corrected-positioning dental panoramic image may be reconstructed using a standard curve, which has been non-rigidly transformed.

Thus, the actual positioning of the patient's head during the panoramic scan is estimated in order to reconstruct one or more dental panoramic images, which are based on the estimated positioning rather than on a predefined expected positioning.

Fig. 2 shows a flow chart of a method for producing a dental panoramic image according to some embodiments.

In step S21, x-ray frame data are recorded during a panoramic scan of a patient, for example using a CBCT scanner as shown in figs. 4A and 4B. In other embodiments, a different type of x-ray dental scanner may be used.

In step S22, at least part of the x-ray frame data is processed prior to being provided as image data input indicative of patient positioning during the panoramic scan. The processing comprises constructing an initial dental panoramic image from the recorded x-ray frame data. The initial dental panoramic image may be reconstructed using a nominal standard curve.

In step S23, the initial dental panoramic image is provided as image data input to a trained machine learning model.

In step S24, the trained machine learning model generates a positioning parameter set based on the received image data input. The positioning parameter set is representative of the position of the patient's head during the panoramic scan.

In step S25, one or more corrected-positioning dental panoramic images are reconstructed based on x-ray frame data recorded during the panoramic scan and on the positioning parameter set. A corrected-positioning dental panoramic image may be reconstructed using a standard curve, which has been rigidly transformed to correspond to an estimated positioning of the patient's dental curve due to the patient's head being e.g. tilted (looking up or down), and/or rotated (head turned), and/or translated. In some embodiments, a corrected-positioning dental panoramic image may be reconstructed using a standard curve, which has been non-rigidly transformed.

Thus, the actual positioning of the patient's head during the panoramic scan is estimated in order to reconstruct one or more dental panoramic images, which are based on the estimated positioning rather than on a predefined expected positioning.

In optional step S26, one or more corrected-positioning dental panoramic images are provided to a user interface and displayed to a user. The process may then await user input such as an adjustment input.

In optional step S27, adjustment input representative of a manually adjusted positioning parameter set, i.e. a positioning parameter set, which is based on the adjustment input from a user, is received.

In optional step S28, one or more manually-adjusted dental panoramic images are reconstructed based on x-ray frame data recorded during the panoramic scan and on the manually adjusted positioning parameter set.

The initial dental panoramic image may be reconstructed with a lower resolution compared to that of the later reconstructed dental panoramic images, where the later reconstructed dental panoramic images may refer to the corrected-positioning dental panoramic images and/or the manually-adjusted dental panoramic images.

The resolution of a reconstructed dental panoramic image depends on how densely the image curve is sampled, i.e. at how many points on the curve is sampled. The curve used for reconstruction of a panoramic image is a 2D curve in the 3D space. The density of the sampling defines the resolution of the resulting reconstructed image, i.e. the distance between the points is the size of each pixel in the reconstructed image. As an example, the later reconstructed dental panoramic images may be sampled with approximately 4000 evenly distanced points. For the initial reconstruction, i.e. the reconstruction of the initial dental panoramic image, one may advantageously choose to sample the curve with much fewer points to provide an image of much lower resolution (larger pixels). In other words, the initial panoramic image is reconstructed in a resolution p by q, while the later reconstructed panoramic images may be reconstructed in a resolution m by n, where p<m and q<n. In some cases, p is chosen to be much smaller than m, and q is chosen to be much smaller than n, for example p may be e.g. 1/2 or 1/3 or ¼ of m, and q likewise may be e.g. 1/2 or 1/3 or ¼ of n. When referring to a sampling with a number of points, the number of points refers to the total number of points. In some embodiments, an initial dental panoramic image is reconstructed using a sampling with between 128 and 2400 points, such as between 200 and 2048 points, such as between 256 and 1500 points, such as between 300 and 1024 points, such as between 512 and 1024 points. In some embodiments, the corrected-positioning dental panoramic images and/or the manually-adjusted dental panoramic images are reconstructed using a sampling with between 2500 and 5000 points, such as between 2750 and 4750 points, such as between 3000 and 4500 points, such as between 3250 and 4250 points, such as between 3500 and 4000 points.

Reconstructing the initial dental panoramic image with a lower resolution has at least two advantages: the initial dental panoramic image can be processed faster by the machine learning model and the lower resolution will mean that the main anatomical structures, such as e.g. mandible, maxilla, etc., are most visible. The main anatomical structures are significant markers for estimation of the patient's head position, whereas the finer structures, such as e.g. trabecular structures inside the bones, which are important for the diagnosis and treatment planning, are much less relevant for the purpose of head position estimation. At lower resolution the finer structures disappear or become much less visible, which means they cause less "noise" for the position estimation. Thus, in some embodiments, the initial dental panoramic image has a lower resolution compared to the resolution of the one or more corrected-positioning dental panoramic images. Further, the one or more manually-adjusted dental panoramic images may be reconstructed with a resolution similar to that of the one or more corrected-positioning dental panoramic images.

Fig. 3 shows a flow chart of a computer-implemented method for producing a dental panoramic image according to some embodiments.

In step S31, one or more images recorded during a panoramic scan of a patient are received,

In optional step S32, the received one or more images are processed.

In step S33, image data input based on the recorded one or more images is provided to a trained machine learning model. The image data input is indicative of patient positioning. The image data input may comprise one or more of: one or more optical images, and/ one or more x-ray images, such as one or more projection x-ray images, and/or one or more reconstructed images, such as one or more reconstructed panoramic images.

In step S34, a positioning parameter set is generated by the trained machine learning model. The positioning parameter set is based on the received image data input and the positioning parameter set is representative of the position of a patient's head during a panoramic scan,

In step S35, one or more corrected-positioning dental panoramic images are reconstructed based on x-ray frame data recorded during the panoramic scan and on the positioning parameter set.

Figs. 4A and 4B show a perspective view of parts of an x-ray imaging system according to some embodiments.

The x-ray imaging system comprises a device in the form of a CBCT scanner 40, where the CBCT scanner comprises an x-ray detector 41, and an x-ray tube component 47 comprising an x-ray radiation source 42 positioned opposite the x-ray detector. The x-ray detector is configured to convert x-rays to digital electric signals pixel by pixel and to output the electric signal as x-ray frame data. In fig. 4A, a patient is positioned in the scanner with only the patient's head 46 being shown in the figure. The detector and/or the x-ray tube component are able to turn substantially around a full circle around a patient's head 46 when the patient is positioned in the scanner. The detector and x-ray tube component are coupled to a support, which in the examples of figs. 4A and 4B is a ring 45. In other systems, the support may have another shape, i.e. may not be ring-shaped. For example, the support may be a straight arm that is rotatable and translatable. In some systems, the detector 41 and x-ray tube component 47 may be fixed to the ring 45 such that they rotate together with the ring.

The system may comprise a chin rest 43 for the patient to rest his/her chin and/or a grip 48, which the patient may grip to steady themselves, while positioned in the scanner. The chin rest 43 and grip 48 are supported within the patient area of the scanner by a support rod 49.

Also comprised in the scanner 40 are four cameras 44, which are comprised in the x-ray tube component 47. The four cameras are more clearly visible in fig. 4B, where they are also all provided with a reference number. The four cameras 44 are configured to each record one or more optical images of the patient during a panoramic scan. In some scanners, one or more cameras may additionally, or alternatively, be positioned in the housing of the detector, and/or on the support, e.g. the ring 45, and/or on the support rod 49. A camera positioned on a part that rotates during the panoramic scan may be configured to capture multiple optical images showing different views of the patient during the scan. For example, frontal views and/or side views of the patient.

The cameras may be mounted with a known geometrical relationship to the detector 41 and radiation source 42. Such a known geometrical relationship may be centred near or in the centre of the ring 45, since the patient will usually be positioned underneath the centre of the ring 45. The one or more cameras are configured to record one or more optical images of the patient, while the scanner acquires x-ray frame data, i.e. substantially simultaneously with the scan.

Following acquisition of the panoramic x-ray data, one or more of the optical images recorded during the scan are provided as image data input to a trained machine learning model, which outputs a positioning parameter set representative of the position of the patient's head during the panoramic scan as described herein.

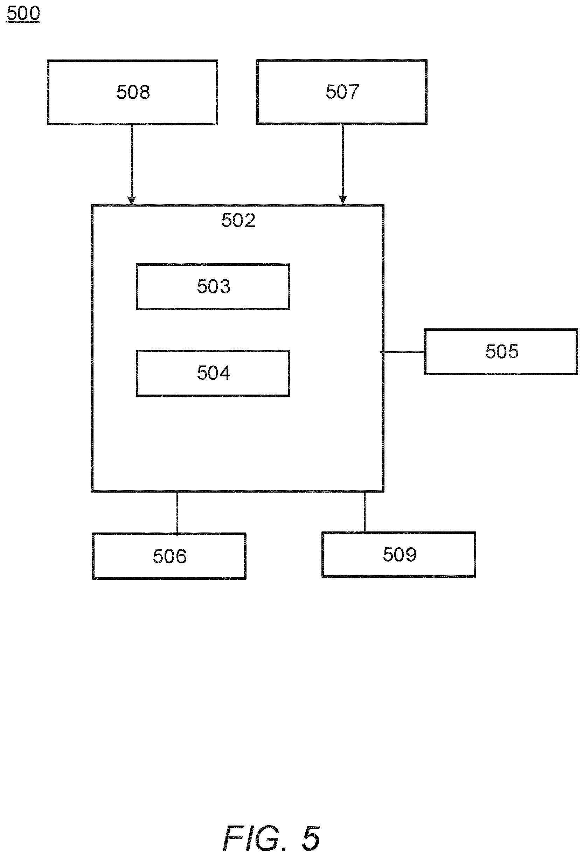

Fig. 5 shows a schematic of at least part of an x-ray imaging system, generally designated by reference numeral 500, according to some embodiments. The system 500 comprises a data processing system 502 comprising a data storage device 504, such as a computer readable medium, and a data processor 503.

In some systems, the data processing system 502 may be a local processing system, an external processing system, or a combination of a local and an external processing system. Some or all of the processing steps described herein, such as reconstruction of dental panoramic images, may be performed by a local data processing system, or some or all of the processing steps may be performed by an external data processing system, or the processing steps may be distributed between a local processing system and an external processing system. A local processing system may be one that is part of or directly coupled to a device, which houses the x-ray detector providing x-ray frame data.

The data processor 503 may be a customized data processor, but may alternatively or additionally comprise a general- or special-purpose programmable microprocessor unit, such as a central processing unit (CPU) of a computer or of another data processing system, an application specific integrated circuits (ASIC), a programmable logic arrays (PLA), a field programmable gate array (FPGA), a special purpose electronic circuit, etc., or a combination thereof. The data processor 503 may be configured to execute reconstruction algorithms. The data storage device 504 is configured for storing program code, such as e.g. the trained machine learning model, and may be additionally configured for storing received x-ray frame data, reconstructed images, positioning parameter set, etc. Examples of suitable data storage devices include a hard disk, an EPROM, etc. The data processor 503 further comprises a trained machine learning model, such as a trained neural network. The trained machine learning model may be comprised in a local processing system, or in an external processing system, or it may be a distributed machine learning model running in series or parallel on both a local and an external processing system. The x-ray imaging system 500 further comprises a visual display unit 505 and one or more input devices 506, such as a computer keyboard, and/or a computer mouse, and/or a touch screen, for entering data and activating virtual buttons visualized on the visual display unit 505. The visual display unit 505 may be a computer screen. In some embodiments, the visual display unit 505 is a touch screen and the input device may be comprised in the visual display unit.

The data processing system 502 is configured to receive x-ray frame data from an x-ray detector 507 and may, optionally, be configured to receive one or more images from one or more image sensors 508, such as from one or more cameras.

The received data and image(s) may be stored in the data storage device 504. The data processing system is configured to provide an image data input based on one or more recorded images. The image data input may also be stored in the data storage device 504 before being provided to the trained machine learning model comprised in the data processor 503.

The trained machine learning model is trained to determine the position of at least part of the patient, such as the position of the patient's head, and it does so based on the received image data input. The trained machine learning model generates an output, a positioning parameter set, which is representative of the position of the patient's head. The data processor 503 is configured to reconstruct one or more corrected-positioning dental panoramic images based on x-ray frame data recorded during the panoramic scan and on the positioning parameter set.

The data processing system may be configured to provide the positioning parameter set, e.g. a set of parameters that define the patient positioning, as well as the one or more reconstructed corrected-positioning panoramic images to the visual display unit, where they may be displayed to a user. Via the one or more input devices 506 the data processing system 502 may receive adjustment input representative of a manually adjusted positioning parameter set, i.e. a positioning parameter set, which is based on the adjustment input from a user. The data processor may be configured to reconstruct one or more manually-adjusted dental panoramic images based on x-ray frame data recorded during the panoramic scan and on the manually adjusted positioning parameter set. The manually adjusted positioning parameter set and the manually-adjusted dental panoramic images may be provided to the visual display unit 505, where they may be displayed to a user, and where the manually adjusted positioning parameter set may be further adjusted manually by a user. The process of manually adjusting the positioning parameter set and reconstructing a manually-adjusted dental panoramic images based thereon may be repeated multiple times.

The data processing system 502 may reconstruct multiple dental panoramic images and in each of the multiple images, one or more of the parameters comprised in the positioning parameter set may be augmented in each of the multiple images. For example, multiple dental panoramic images could be reconstructed each being slightly offset along the patient's anterior/posterior axis. The multiple dental panoramic images based on augmented positioning parameters may be provided to the visual display unit 505. The one or more input devices may comprise e.g. a button, or slider, or the like, which is configured to provide the user with a way to change which of the multiple dental panoramic images is displayed or is in focus. For example, the visual display unit 505 may display multiple panoramic images, one at a time, where each of the images is slightly offset along the patient's anterior/posterior axis compared to the previous and following images. This would allow the user to see the multiple dental panoramic images in a manner where the focus is shifted in the anterior/posterior direction, for example to refocus on the roots/crown of an anterior tooth oriented somewhat perpendicular to the individual images.

The x-ray imaging system 500 also comprises a transmission unit 509 which allows data to be sent to other systems, for example for further processing. The transmission unit may be a connection to the internet, such as a wired or wireless connection, via which the data, such as e.g. reconstructed one or more corrected-positioning dental panoramic images, may be transmitted.

## Claims

1. **A method for producing a dental panoramic image**, the method comprising:
- recording one or more images during a panoramic scan of a patient,
- providing an image data input to a trained machine learning model, the image data input being based on the recorded one or more images and the image data input being indicative of patient positioning during the panoramic scan,
- generating a positioning parameter set by the trained machine learning model, the positioning parameter set being based on the received image data input, where the positioning parameter set is representative of the position of the patient's head during the panoramic scan,
- reconstructing one or more corrected-positioning dental panoramic images based on x-ray frame data recorded during the panoramic scan and on the positioning parameter set.

2. The method according to claim 1, wherein the one or more recorded images comprises one or more of: one or more optical images, and/or one or more x-ray images, such as one or more projection x-ray images.

3. The method according to any of claims 1 or 2, wherein the image data input comprises one or more of: one or more optical images, one or more x-ray images, such as one or more projection x-ray images, and/or one or more reconstructed images, such as one or more reconstructed panoramic images.

4. The method according to any of the previous claims, wherein the position of the patient's head during the panoramic scan is defined relative to a pre-defined coordinate system, and wherein the pre-defined coordinate system is a pre-defined head positioning or a pre-defined global coordinate system.

5. The method according to claim 4, wherein the positioning parameter set is indicative of a rigid transformation, i.e. rotation and/or translation, of the pre-defined coordinate system and/or of a non-rigid transformation, such as scaling and/or shear, of the pre-defined coordinate system.

6. The method according to any of the previous claims, wherein the method further comprises:
- generating an initial dental panoramic image based on x-ray frame data recorded during the panoramic scan,
and the step of providing image data input to a trained machine learning model further comprises providing the initial dental panoramic image as image data input to the trained machine learning model.

7. The method according to claim 6, wherein the initial dental panoramic image has a lower resolution compared to the resolution of the one or more corrected-positioning dental panoramic images.

8. **An x-ray imaging system** comprising:
- an x-ray detector configured to convert x-rays to digital electric signals pixel by pixel and to output the electric signal as x-ray frame data,
- a data storage unit configured to store the x-ray frame data outputted from the X-ray detector during a scan of a patient,
- a data processing system,
the x-ray imaging system being configured to execute the steps of any of the preceding claims.

9. **An x-ray imaging system** comprising:
- an x-ray detector configured to convert x-rays to digital electric signals pixel by pixel and to output the electric signal as x-ray frame data,
- a data storage device configured to store the x-ray frame data outputted from the x-ray detector during a panoramic scan of a patient,
wherein the x-ray imaging system further comprises a data processing system comprising a trained machine learning model configured to receive image data input indicative of patient positioning during the scan of the patient, the image data input being based on one or more images recorded during the panoramic scan,
the trained machine learning model being configured to generate a positioning parameter set based on the received image data input, where the positioning parameter set is representative of the position of the patient's head during the panoramic scan, and
wherein the data processing system is further configured to reconstruct one or more corrected-positioning dental panoramic images based on x-ray frame data recorded during the panoramic scan and on the positioning parameter set.

10. The x-ray imaging system according to claim 9, wherein the one or more recorded images comprises one or more of: one or more x-ray images, such as one or more projection x-ray images, and/or
wherein the x-ray imaging system further comprises one or more cameras and the recorded one or more images comprises one or more optical images.

11. The x-ray imaging system according to any of claims 9 or 10, wherein the image data input comprises one or more of: one or more x-ray images, such as one or more projection x-ray images, and/or one or more reconstructed images, such as one or more reconstruction panoramic images, and/or
wherein the x-ray imaging system further comprises one or more cameras and the image data input comprises one or more optical images.

12. The x-ray imaging system according to any of claims 9 - 11, wherein the position of the patient's head during the panoramic scan is defined relative to a pre-defined coordinate system, and wherein the pre-defined coordinate system is a pre-defined head positioning or a pre-defined global coordinate system, and wherein the positioning parameter set is indicative of a rigid transformation, i.e. rotation and/or translation, of the pre-defined coordinate system and/or of a non-rigid transformation, such as scaling and/or shear, of the pre-defined coordinate system.

13. The x-ray imaging system according to any of claims 9-12, wherein the data processing system is configured to generate an initial dental panoramic image based on x-ray frame data recorded during the panoramic scan, and wherein the image data input provided to the trained machine learning model comprises the initial dental panoramic image.

14. The x-ray imaging system according to claim 13, wherein the initial dental panoramic image has a lower resolution compared to the resolution of the one or more corrected-positioning dental panoramic images.

15. **A computer-implemented method for producing a dental panoramic image,** the method comprising the steps:
- receiving one or more images recorded during a panoramic scan of a patient,
- providing an image data input to a trained machine learning model, the image data input being based on the recorded one or more images and the image data input being indicative of patient positioning during the panoramic scan,
- generating a positioning parameter set by the trained machine learning model, the positioning parameter set being based on the received image data input, where the positioning parameter set is representative of the position of a patient's head during a panoramic scan,
- reconstructing one or more corrected-positioning dental panoramic images based on x-ray frame data recorded during the panoramic scan and on the positioning parameter set.
